# FASCICULE DE BREVET EUROPEEN

(11) **EP 1 202 764 B1**
(45) Date de publication et mention de la délivrance du brevet: **15.09.2004**
(21) Numéro de dépôt: 00949591.2
(22) Date de dépôt: 30.06.2000
(51) Int. Cl.: A61M 5/30

(54) **SERINGUE SANS AIGUILLE FONCTIONNANT AVEC UN GENERATEUR D'ONDE DE CHOC A TRAVERS UNE PAROI**
NADELLOSE SPRITZE MIT EINEM DURCH EINE TRENNWAND WIRKENDEN STOSSWELLENGENERATOR
NEEDLELESS SYRINGE OPERATING WITH AN IMPACT WAVE GENERATOR THROUGH A WALL

(30) Priorité: 16.07.1999 FR 9909255
(43) Date de publication de la demande: 08.05.2002
(73) Titulaire: CROSSJECT, 75181 Paris Cedex 04 (FR)
(72) Inventeur: ALEXANDRE, Patrick, F-70100 Gray (FR); BRUNET, Pierre, F-91510 Lardy (FR); CAGNON, Brigitte, F-91610 Ballancourt (FR); MIKLER, Claude, F-21000 Dijon (FR)
(74) Mandataire: Waligorski, Carol
(86) Numéro de dépôt international: PCT/FR2000/001848
(87) Numéro de publication internationale: WO 2001/005451

(56) Documents cités:
- WO-A-94/24263
- WO-A-96/20022
- WO-A-96/25190
- WO-A-99/04838

## Description

La présente invention est dans le domaine des seringues sans aiguille utilisées pour les injections intradermique, sous-cutanée ou intramusculaire de divers principes actifs à usage thérapeutique pour la médecine humaine ou vétérinaire.

De nombreux types de seringues sans aiguille pour injecter des principes actifs liquides sont connus depuis 1945. Dans ces dispositifs le principe actif liquide est refoulé, à travers une buse comportant au moins un orifice, par un piston ou par la déformation d'une enveloppe souple contenant ledit liquide, ladite enveloppe souple étant reliée à la buse. La pression d'injection est obtenue soit par la détente d'un ressort initialement comprimé, soit par la détente d'un gaz stocké sous pression, comme cela est décrit dans le brevet US 3,788,315. D'autres dispositifs d'injection sous cutanée utilisent une charge pyrotechnique génératrice de gaz pour propulser un piston qui refoule le liquide à injecter et le brevet US 3,802,430 illustre une telle technique de génération de gaz propulsif.

Pour l'injection de principes actifs solides, sous forme de poudre sèche, les seringues sans aiguille utilisent différents moyens pour accélérer les particules dudit principe actif et il faut citer la demande de brevet WO 94/24263 qui décrit une seringue sans aiguille dont les particules du principe actif sont entraînées par un écoulement gazeux à grande vitesse par la détente, à travers une tuyère, d'un gaz comprimé. Malgré l'utilisation d'une longue tuyère il y a production d'un nuage dispersé de particules qui laisse supposer une faible biodisponibilité. Il faut de plus, par des dispositifs annexes, dissiper les effets de souffle et de bruit du jet gazeux. Enfin la fiabilité du système dépend de celle du dispositif de stockage des gaz sous pression.

Le document WO 96/25190 divulgue un dispositif qui montre l'ensemble des caractéristiques du préambule de la revendication 1.

Dans un autre domaine technique le brevet US 4,945,050 décrit le principe d'un appareil de laboratoire pour bombarder une culture cellulaire avec des microparticules métalliques enduites de diverses substances biologiques à des fins de transfection génique. Différents moyens sont notamment utilisés pour transférer l'énergie cinétique d'un projectile impactant une barrière, aux particules disposées sur le projectile ou sur cette barrière et faire pénétrer ces particules dans la culture cellulaire. Cet appareil de laboratoire fonctionne sous vide et utilise des microparticules neutres très denses pour augmenter l'énergie cinétique (microparticules d'or ou de tungstène), ce qui exclut toute application ou toute transposition comme seringue sans aiguille, d'autant plus qu'il produit un nuage de , particules pour atteindre le plus grand nombre possible de cellules.

Un des buts de l'invention est de surmonter les inconvénients concernant les seringues sans aiguille permettant l'injection de principe actif pulvérulent. Un autre but de la présente invention est de mettre à disposition une seringue plus universelle qui peut être utilisée pour l'injection non seulement de principes actifs pulvérulents mais aussi de principes actifs liquides, ou en suspension dans un liquide, ou sous forme de gel. La transformation du volume initial du principe actif et sa mise en forme effilée au moment de la pénétration dans la peau sont obtenues par un effet de retournement et de focalisation qui a été découvert et qui a été observé aussi bien sur des liquides, des gels ou des poudres.

La présente invention est définie par la revendication 1 et concerne une seringue sans aiguille pour l'injection de principe actif à usage thérapeutique, comprenant de l'amont vers l'aval, un système propulsif, le principe actif et un guide d'application de ladite seringue sur la peau du sujet à traiter, cette seringue étant telle que, d'une part, le système propulsif est constitué par un dispositif générateur d'une onde de choc et, d'autre part, le principe actif est disposé dans au moins une cavité borgne de la face aval d'une barrière prolongée par le guide d'application. En effet, la barrière présente deux faces opposées, l'une, amont, située du côté du dispositif générateur d'une onde de choc et l'autre, aval, située du côté du guide d'application.

La face amont de la barrière, est sensiblement plane et transversale. La face aval de la barrière fixe, comporte au moins une cavité borgne, cette cavité ayant donc une ouverture sur la seule face aval de la barrière fixe et ne débouchant pas sur la face amont, en ménageant une épaisseur suffisante pour résister à l'onde de choc.

Avantageusement, le dispositif générateur de l'onde de choc produit une onde de choc plane sur la face amont de la barrière fixe. L'onde de choc plane produite sur la face amont de la barrière fixe se propage à travers cette barrière, et éjecte violemment le principe actif depuis chacune des cavités où il était disposé. De façon inattendue, le principe actif ainsi accéléré à très grande vitesse peut se regrouper sous forme d'un jet central de faible diamètre qui va pénétrer ensuite dans la peau du sujet. Chaque jet correspond à une cavité et présente une forme effilée à son extrémité aval, dès que la distance parcourue par ce jet correspond à quelques diamètres de la barrière fixe. Il faut préciser que le phénomène d'éjection sous forme de jets est pleinement efficace lorsque l'onde de choc est plane au moment où elle atteint les cavités. Ceci n'exclut pas que l'onde de choc produite sur la face amont peut ne pas être rigoureusement plane et présenter une légère courbure qui sera effacée lors de la propagation dans la barrière.

Ce phénomène de conformation en jet effilé du principe actif à partir du principe initialement stocké sous forme globulaire, dans tout ou partie d'une cavité soumise à une onde de choc, est assimilable aux jets de matière qui résultent de la détonation de chargements explosifs présentant une face aval concave recouverte par une feuille métallique. De tels chargements explosifs dits « charges creuses.» permettent d'obtenir des dards effilés à température élevée qui sont propulsés à des vitesses de l'ordre de 8000 mètres par seconde et sont capables de percer des blindages pouvant atteindre 1 mètre d'épaisseur. Le phénomène mis en jeu dans la présente invention est cependant de nature tout à fait différente puisque le principe actif ne doit pratiquement pas subir d'élévation de température et que des vitesses de propulsion de l'ordre de 600 à 1000 mètres par seconde sont tout à fait suffisantes pour permettre une injection au travers des épidermes. Contrairement aux chargements explosifs formant un jet central de matière, l'explosif ne doit pas être au contact de la matière à éjecter et doit être séparé par une barrière résistante qui assure une bonne propagation de l'onde de choc, cette barrière pouvant, par exemple, être faite en aluminium ou en acier. Du fait de la vitesse limitée d'éjection du jet de principe actif, il a été découvert que non seulement l'onde de choc pouvait être obtenue par une faible quantité d'explosif dont la détonation est amorcée par un microdétonateur, mais que cette onde de choc pouvait être obtenue par d'autres moyens tels que la propulsion d'une masselotte qui percute la face amont de la barrière fixe.

Avantageusement, pour obtenir un jet effilé, chaque cavité borgne de la barrière fixe présente une section transversale d'ouverture au moins égale à toute section transversale de cette cavité et, préférentiellement, chaque cavité présente une forme de révolution autour d'un axe parallèle à la direction de propagation de l'onde de choc, dans le but de favoriser la formation d'un jet parfaitement aligné sur l'axe de ladite cavité.

Préférentiellement, une cavité peut avoir, par exemple, une forme hémisphérique, conique ou tronconique ou être constituée par une combinaison de ces profils.

Selon une variante de réalisation, plusieurs cavités sont réparties sur la face aval de la barrière. Avantageusement, ces cavités pouvant avoir des formes différentes, sont régulièrement réparties sur ladite surface.

Le principe actif est déposé avantageusement au fond de la cavité, et, préférentiellement, il remplit toute la cavité jusqu'à affleurer la partie plane de la face aval où il est retenu, par exemple, au moyen d'un film de faible épaisseur. En effet, des simulations numériques ont montré qu'avec une telle configuration de remplissage, la dispersion diamétrale du jet est minimum.

Le matériau utilisé pour la barrière fixe est choisi dans le groupe des matériaux ne donnant pas lieu au phénomène d'écaillage sous l'action d'une onde de choc, comme par exemple les métaux. Il est également choisi en fonction de sa densité et de son impédance acoustique, autrement dit de son aptitude à transmettre l'onde de choc. Il est notamment déterminé par rapport à la vitesse que l'on souhaite communiquer au jet.

Dans une première réalisation, l'onde de choc sur la face amont de la barrière fixe est produite par l'impact sur la dite face amont d'une masselotte de forme appropriée, accélérée par un dispositif annexe. Avantageusement le diamètre de ladite masselotte est tel que l'air compris entre la masselotte et la barrière fixe est chassé sans freiner ladite masselotte guidée par des moyens appropriés. L'accélération de la masselotte se fait, soit par la détente d'un ressort comprimé, soit par la combustion d'une charge pyrotechnique, soit par la détente de gaz comprimé.

Dans une seconde réalisation, l'onde de choc plane sur la face amont de la barrière fixe est produite par un générateur d'onde de choc comportant un chargement pyrotechnique détonant. Avantageusement, celui-ci comprend un feuillet d'explosif adjacent à la face amont, ledit feuillet d'explosif étant amorcé en détonation soit ponctuellement soit sur tout ou partie de sa surface par un microdétonateur. Ce feuillet d'explosif a un diamètre sensiblement égal à celui de la barrière fixe et comprend quelques dizaines de milligrammes d'un explosif tel que le TNT ou d'un explosif composite à grande vitesse de détonation.

Le guide d'application a une longueur telle qu'il permet au principe actif pendant son éjection vers la peau de se rassembler sous forme d'un jet effilé. Avantageusement, la longueur du guide d'application est comprise entre 1 et 8 fois le diamètre de la barrière fixe et, préférentiellement, entre 2 et 5 fois le diamètre de la barrière fixe.

Avantageusement, le guide d'application comporte un système amortissant qui peut être réduit à un simple bourrelet souple situé à son extrémité en appui sur la peau du sujet à traiter, ou consister en la réalisation d'un guide d'application télescopique avec un ressort interne.

La présente invention résout bien les problèmes posés et permet, par exemple, d'obtenir, pour une barrière de 5mm de diamètre et de 2mm d'épaisseur, des jets de diamètres échelonnés entre 0,12mm et 1,6mm lorsque des cavités ogivales, sensiblement hémisphériques ou coniques sont utilisées, mais dans le cas de cavités coniques notamment, il est avantageux que la face aval du principe actif soit plane et ne dépasse pas 3 à 4mm de diamètre.

Pour un diamètre de barrière de 5 millimètres, une épaisseur de 3 millimètres et une cavité hémisphérique d'un millimètre de rayon, en utilisant une pastille d'explosif d'un diamètre de 3 millimètres et d'épaisseur 1 millimètre, il est possible d'obtenir un jet présentant un diamètre maximal de 0,7 millimètre avec une vitesse d'éjection de 630 mètres par seconde.

L'effet majeur recherché par le dispositif d'éjection selon l'invention est un effet atténué dit de « charge creuse » se traduisant par la formation d'un jet effilé, composé par les particules de matière à expulser, et animé, suivant son axe, d'une vitesse très élevée lui procurant une grande force de pénétration. Les caractéristiques de ce jet, à savoir sa forme, sa longueur, sa dispersion et sa vitesse de déplacement, sont fonction de la nature et du positionnement du générateur d'ondes planes, du matériau constitutif de la pièce servant de barrière et de la géométrie des cavités de la face aval servant à loger les particules. A une échelle moindre, la forme générale du volume constitué par les particules dans chaque cavité joue également un rôle.

De façon avantageuse, sous l'effet de la charge pyrotechnique, la barrière, bien qu'ayant été déformée, reste fixée dans la seringue à sa position d'origine. Selon un autre mode de réalisation de l'invention, face à la même sollicitation, la barrière est déplacée mais reste piégée dans la seringue sans possibilité d'être expulsée.

Préférentiellement, le guide d'application est constitué par un corps cylindrique creux dont la section est voisine de celle constituée par la face aval de la barrière et dont l'axe est perpendiculaire à ladite face. Ce guide est particulièrement recommandé pour optimiser les conditions de réalisation d'un impact perpendiculaire à ladite surface.

Selon un premier mode de réalisation de l'invention, les particules forment un amas pulvérulent dans chaque cavité. Ces particules sont maintenues dans leur logement par capillarité, par électricité statique, par un état de surface adhésif ou par une peau recouvrant chaque cavité. Enfin, tout moyen d'adhérence peut-être retenu à condition qu'il n'interfère pas au niveau de la formation du jet.

Selon un second mode de réalisation de l'invention, les particules sont liées entre elles par un liquide fluide, visqueux ou sous forme gel. Par rapport à ce liquide, les particules de principe actif peuvent se retrouver soit en suspension, soit en solution

De façon avantageuse, le déclencheur peut-être un bouton poussoir provoquant l'initiation du microdétonateur par percussion.

Le dispositif, selon l'invention présente l'avantage d'être performant tout en étant de conception simple et légère, et peu encombrant. En effet, la technique impliquant la formation d'un jet sur le modèle charge creuse, permet de projeter des particules sous forme concentrée et à très haute vitesse à partir d'un dispositif faisant intervenir un nombre de pièces restreint, réalisées avec des matériaux légers et agencées entre elles de façon simple.

De plus, un dimensionnement rapide permet d'adapter le dispositif à un large éventail de situations, en jouant notamment sur la section du jet, sa dispersion, sa longueur, sa vitesse et le nombre de jets à expulser.

Par ailleurs, le principe actif n'étant pas mis en mouvement par la détente des gaz, il n'y a pas d'effet de souffle. Il n'y a pas non plus de bruit lié à la détente des gaz à l'extérieur, le bruit ne pouvant provenir que de l'impact de la masselotte sur la barrière ou du fonctionnement de la charge pyrotechnique qui sont internes à la seringue.

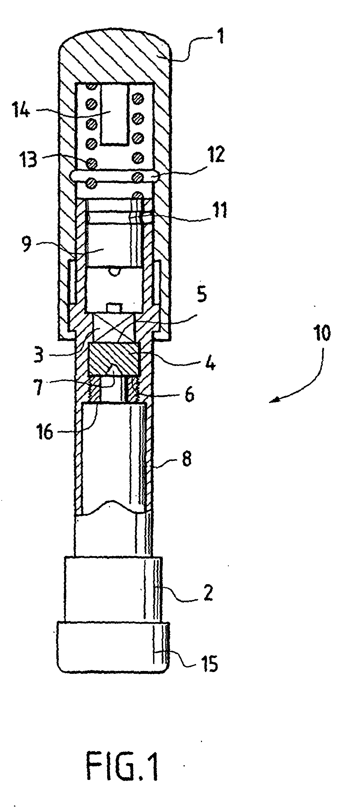

La présente invention va être décrite plus en détail à l'aide de la figure 1, qui représente une vue en coupe partielle d'une seringue selon l'invention, et qui peut être utilisée pour décrire deux générateurs d'onde de choc.

La figure 1 représente une vue d'une seringue 10 avant utilisation, l'extrémité aval de cette seringue 10 étant encore fermée par un bouchon hermétique 15 qui assure l'asepsie de la partie intérieure du guide d'application 8.

Selon cette figure 1, le guide d'application 8 se prolonge à l'intérieur du tube de déclenchement 1 et comporte, en remontant de l'aval vers l'amont, une bague filetée 16 qui assure le blocage d'une barrière fixe 4 sur un épaulement du guide, cet épaulement ménageant une ouverture centrale dans laquelle est placé le dispositif générateur d'une onde de choc 3 constitué par une pastille d'explosif composite sensible, surmontée par un microdétonateur pouvant être initié par percussion. Dans la prolongation interne du guide d'application 8 peut coulisser une masselotte 9 qui comporte un percuteur à son extrémité aval et une gorge de maintien dans laquelle sont engagées trois billes 11 placées dans des perforations radiales de cette prolongation. Ces billes 11 sont en appui sur la surface interne du tube de déclenchement 1 et immobilisent la masselotte 9 creuse de percussion surmontée par un ressort 13 comprimé entre cette masselotte 9 et le fond du tube de déclenchement 1, ce tube 1 étant retenu en position initiale par un épaulement inférieur interne au contact du guide d'application 8.

Le principe actif pulvérulent remplit une cavité en demi-ellipsoide 7 située sur la face aval 6 de la barrière 4, et ce principe est maintenu au moyen d'un film mince fixé sur la barrière fixe 4 et coincé par la bague filetée 16.

En fonctionnement, après avoir retiré le bouchon 15 et appliqué l'extrémité dégagée du guide d'application 8 au contact de la zone de l'épiderme choisie pour l'injection du principe actif, le tube de déclenchement 1 est pressé pour comprimer le ressort 13 jusqu'à ce que la gorge interne 12 de ce tube 1 arrive au niveau des trois billes 11 qui s'écartent radialement et libèrent la masselotte creuse de percussion 9 dont la pointe aval va percuter le microdétonateur, faisant exploser la pastille explosive au contact de la face amont 5 de la barrière fixe 4. L'onde de choc plane ainsi générée, va atteindre la cavité 7 et provoquer simultanément la rupture du film mince et l'éjection du principe actif selon un phénomène de retournement et de focalisation proche du phénomène mis en oeuvre dans les charges creuses. La masselotte creuse 9, le ressort 13 et le matériau 14 ont alors une fonction d'amortissement des effets arrières de la détonation et le système amortissant 2 du guide d'application 8 atténue l'effet de compression avant, cette sensation de compression annulaire masquant en grande partie la sensation de piqûre résultant de la pénétration du jet effilé de produit actif dans l'épiderme et le derme.

Selon une variante de réalisation pouvant être décrite en se repérant à cette même figure, l'onde de choc plane n'est pas produite par un explosif au contact de la barrière 4 dans laquelle est ménagée la cavité borgne 7. Cette variante non représentée nécessite l'utilisation d'une masselotte pleine qui se prolonge en aval par une panne de frappe cylindrique pouvant s'engager dans l'alésage obtenu par retrait de l'explosif et du détonateur de manière à percuter directement la barrière fixe 4. Dans cette variante le ressort 13 est inutile et le matériau 14 doit être remplacé par un générateur pyrotechnique de gaz pouvant être actionné par un moyen extérieur, le tube de déclenchement 1 étant totalement solidarisé au guide d'application 8 et comportant un fourreau interne déformable au niveau des trois billes 11. En fonctionnement, l'initiation du générateur pyrotechnique de gaz assure une montée en pression de la chambre comprise entre le tube de déclenchement et la masselotte, jusqu'à ce que la pression exercée sur cette masselotte provoque l'enfoncement partiel des trois billes dans le fourreau malléable et libère cette masselotte dont la panne assurera la frappe de la barrière fixe 4 et générera une onde de choc plane permettant d'obtenir la formation d'un jet effilé du produit actif initialement stocké sous forme globulaire dans la cavité borgne 7.

## Revendications

1. Seringue sans aiguille (10), pour l'injection de principe actif comprenant de l'amont vers l'aval, un système propulsif constitué par un dispositif générateur d'une onde de choc, une barrière comportant une face amont (5) et une face aval (6), ladite face aval (6) possédant au moins une cavité borgne (7) dans laquelle est logé le principe actif, et un guide d'application (8) de ladite seringue (10) sur la peau du patient à traiter, **caractérisée en ce que**, d'une part, la barrière (4) est fixe et résistante à l'onde de choc et, d'autre part, ladite barrière (4) assure une bonne propagation de l'onde de choc.

2. Seringue sans aiguille selon la revendication 1 **caractérisée en ce que** la barrière (4) comporte une face amont (5) sensiblement plane et transversale.

3. Seringue sans aiguille selon l'une des revendications 1 ou 2 **caractérisée en ce que**, le dispositif générateur de l'onde de choc (3) produit une onde de choc plane sur la face amont (5) de la barrière fixe (4).

4. Seringue sans aiguille selon la revendication 1, **caractérisée en ce que** chaque cavité borgne (7) présente une section transversale d'ouverture au moins égale à toute section transversale de cette cavité (7).

5. Seringue sans aiguille selon l'une des revendications 1 ou 4, **caractérisée en ce que** chaque cavité (7) présente une forme de révolution autour d'un axe parallèle à la direction de propagation de l'onde de choc.

6. Seringue sans aiguille selon l'une des revendications 1 ou 4 **caractérisée en ce que** plusieurs cavités sont réparties sur la face aval (6) de ladite barrière (4).

7. Seringue sans aiguille selon l'une des revendications 1 à 3 **caractérisée en ce que** l'onde de choc sur la face amont (5) de la barrière fixe (4) est produite par une masselotte venant impacter ladite barrière (4).

8. Seringue sans aiguille selon l'une des revendications 1 à 3 **caractérisée en ce que** le générateur de l'onde de choc (3) sur la face amont (5) de la barrière fixe (4) comporte un chargement pyrotechnique détonant.

9. Seringue sans aiguille selon l'une des revendications précédentes **caractérisée en ce que** la longueur du guide d'application (8) est comprise entre 1 et 8 fois le diamètre de la barrière fixe (4) et, préférentiellement, entre 2 et 5 fois.

10. Seringue sans aiguille selon la revendication 9 **caractérisée en ce que** le guide d'application (8) comporte un système amortissant (2).

## Patentansprüche

1. Nadellose Spritze (10) zum Spritzen eines Wirkstoffs, die von stromaufwärts nach stromabwärts ein Antriebssystem, das aus einer Stoßwellengeneratorvorrichtung besteht, eine eine stromaufwärts liegende Seite (5) und eine stromabwärts liegende Seite (6) aufweisende Barriere, wobei die stromabwärts liegende Seite (6) mindestens einen Hohlraum (7) mit geschlossenem Boden aufweist, in dem der Wirkstoff untergebracht ist, und eine Anwendungsführung (8) für die Spritze (10) auf der Haut des zu behandelnden Patienten aufweist, **dadurch gekennzeichnet, dass** einerseits die Barriere (4) ortsfest und stoßwellenfest ist, und dass andererseits die Barriere (4) eine gute Ausbreitung der Stoßwelle gewährleistet.

2. Nadellose Spritze nach Anspruch 1, **dadurch gekennzeichnet, dass** die Barriere (4) eine im wesentlichen ebene und quer verlaufende, stromaufwärts liegende Seite (5) aufweist.

3. Nadellose Spritze nach einem der Ansprüche 1 oder 2, **dadurch gekennzeichnet, dass** die Stoßwellengeneratorvorrichtung (3) eine ebene Stoßwelle auf die stromaufwärts liegende Seite (5) der ortsfesten Barriere (4) erzeugt.

4. Nadellose Spritze nach Anspruch 1, **dadurch gekennzeichnet, dass** jeder Hohlraum (7) mit geschlossenem Boden einen Öffnungsquerschnitt aufweist, der mindestens gleich jedem Querschnitt dieses Hohlraums (7) ist.

5. Nadellose Spritze nach einem der Ansprüche 1 oder 4, **dadurch gekennzeichnet, dass** jeder Hohlraum (7) eine um eine Achse parallel zur Ausbreitungsrichtung der Stoßwelle drehsymmetrische Form aufweist.

6. Nadellose Spritze nach einem der Ansprüche 1 oder 4, **dadurch gekennzeichnet, dass** mehrere Hohlräume auf der stromabwärts liegenden Seite (6) der Barriere (4) verteilt sind.

7. Nadellose Spritze nach einem der Ansprüche 1 bis 3, **dadurch gekennzeichnet, dass** die Stoßwelle auf die stromaufwärts liegende Seite (5) der ortsfesten Barriere (4) durch einen Schlagbolzen erzeugt wird, der auf die Barriere (4) aufprallt.

8. Nadellose Spritze nach einem der Ansprüche 1 bis 3, **dadurch gekennzeichnet, dass** der Generator der Stoßwelle (3) auf die stromaufwärts liegende Seite (5) der ortsfesten Barriere (4) eine explodierende pyrotechnische Ladung aufweist.

9. Nadellose Spritze nach einem der vorhergehenden Ansprüche, **dadurch gekennzeichnet, dass** die Länge der Anwendungsführung (8) zwischen 1 und 8 mal so groß wie der Durchmesser der ortsfesten Barriere (4), und vorzugsweise zwischen 2 und 5 mal so groß ist.

10. Nadellose Spritze nach Anspruch 9, **dadurch gekennzeichnet, dass** die Anwendungsführung (8) ein Dämpfungssystem (2) aufweist.

## Claims

1. A needleless syringe (10) for the injection of active substance, comprising, from upstream to downstream, a propulsive system consisting of a device for generating a shock wave, a barrier comprising an upstream face (5) and a downstream face (6), said downstream face (6) having at least one blind cavity (7), in which there is accommodated the active substance, and a guide (8) for application of said syringe (10) onto the skin of the patient to be treated, **characterised in that**, on the one hand, the barrier (4) is fixed and resistant to the shock wave and, on the other hand, said barrier (4) ensures good propagation of the shock wave.

2. A needleless syringe according to claim 1, **characterised in that** the barrier (4) comprises an upstream face (5) which is substantially planar and transverse.

3. A needleless syringe according to one of claims 1 or 2, **characterised in that** the device for generating the shock wave (3) produces a planar shock wave on the upstream face (5) of the fixed barrier (4).

4. A needleless syringe according to claim 1, **characterised in that** each blind cavity (7) exhibits an opening cross-section which is at least equal to every cross-section of said cavity (7).

5. A needless syringe according to one of claims 1 or 4, **characterised in that** each cavity (7) exhibits a shape generated by rotation about an axis parallel to the direction of propagation of the shock wave.

6. A needleless syringe according to one of claims 1 or 4, **characterised in that** a plurality of cavities are distributed over the downstream face (6) of said barrier (4).

7. A needleless syringe according to one of claims 1 to 3, **characterised in that** the shock wave on the upstream face (5) of the fixed barrier (4) is produced by an inertia block impacting on said barrier (4).

8. A needleless syringe according to one of claims 1 to 3, **characterised in that** the generator of the shock wave (3) on the upstream face (5) of the fixed barrier (4) comprises an explosive pyrotechnic charge.

9. A needleless syringe according to one of the preceding claims, **characterised in that** the length of the application guide (8) is between 1 and 8 times the diameter of the fixed barrier (4) and, preferably, between 2 and 5 times.

10. A needleless syringe according to claim 9, **characterised in that** the application guide (8) comprises a damping system (2).
